# EUROPEAN PATENT APPLICATION

(11) **EP 3 705 051 A1**
(43) Date of publication of application: **09.09.2020**
(21) Application number: 20161774.3
(22) Date of filing: 09.03.2020
(51) Int. Cl.: A61B 8/08

(54) **ULTRASONIC IMAGING APPARATUS AND METHOD OF CONTROLLING THE SAME**

(30) Priority: 08.03.2019 KR 20190026690
(71) Applicant: Samsung Medison Co., Ltd., Hongcheon-gun, Gangwon-do, 25108 (KR)
(72) Inventor: Son, Eunju, 12786 Gyeonggi-do (KR); Kim, Mose, 07431 Seoul (KR); Kim, Jungho, 05267 Seoul (KR); Jin, Gilju, 02828 Seoul (KR)
(74) Representative: Arnold & Siedsma

(57) **Abstract**

An ultrasonic imaging apparatus and a method of controlling the ultrasonic imaging apparatus for obtaining an image of an object by efficiently removing noise from an ultrasonic image obtained using a contrast agent are provided. The ultrasonic imaging apparatus includes a display; and a controller configured to derive a difference between ultrasonic image signals including contrast agent signals obtained in a frame before and after at least one viewpoint, and to form an image of an object based on the difference between the ultrasonic image signals over time corresponding to the frame before and after at least one viewpoint and output the image of the object to the display.

## Description

### CROSS-REFERENCE TO RELATED APPLICATION(S)

This application is based on and claims priority under 35 U.S.C. §119 to Korean Patent Application No. 10-2019-0026690, filed on March 8, 2019 in the Korean Intellectual Property Office, the disclosure of which is incorporated by reference in its entirety.

### TECHNICAL FIELD

The disclosure relates to an ultrasonic imaging apparatus for obtaining an image of an object using a contrast agent and a method of controlling the ultrasonic imaging apparatus.

### BACKGROUND

Recently, an ultrasonic contrast agent (UCA) image using ultrasonic contrast agents (UCAs) has been widely used for diagnosis of various lesions. The UCA is injected into a human body, and the injected UCA circulates in the human body. The UCAs, composed of microbubbles, may act as strong reflectors of ultrasonic signals. Therefore, the ultrasonic signal reflected from the microbubbles is displayed as a strong signal compared to a general tissue in an ultrasonic image. As such, after the UCA is injected into blood flow, a method of identifying blood vessel information in detail by detecting a strong UCA signal in the blood flow is a general UCA imaging technique.

For the UCA image, a general tissue signal in the image is minimized, but a transmission / reception technique for highlighting the UCA signal is mainly used, and thus the signal change according to a flow of the UCA can be observed more clearly. The microbubbles of the UCA are usually less than red blood cells and flow through most blood vessels in the blood flow of the human body. When using the UCA image, there is an advantage that it is possible to identify signals from micro blood flow that is not identified by general ultrasonic.

In recent years, it has been used to diagnose infertility / fertility such as malformations of the uterus and blockage of fallopian tubes by injecting into the uterine lumen as well as blood vessels and to diagnose urinary ureter reflux by injecting it into the bladder.

### SUMMARY

Therefore, it is an aspect of the disclosure to provide an ultrasonic imaging apparatus for obtaining an image of an object by efficiently removing noise from an ultrasonic image obtained using a contrast agent, and a method of controlling the ultrasonic imaging apparatus.

Additional aspects of the disclosure will be set forth in part in the description which follows and, in part, will be obvious from the description, or may be learned by practice of the disclosure.

In accordance with an aspect of the disclosure, an ultrasonic imaging apparatus includes a display; and a controller configured to derive a difference between ultrasonic image signals including contrast agent signals obtained in a frame before and after at least one viewpoint, and to form an image of an object based on the difference between the ultrasonic image signals over time corresponding to the frame before and after at least one viewpoint and output the image of the object to the display.

The at least one viewpoint may include a viewpoint at which a contrast agent is injected into the object.

The controller may be configured to form a noise removing filter based on the difference between the ultrasonic image signals corresponding to the viewpoint at which the contrast agent is injected into the object.

The controller may be configured to derive position information of a difference signal of the ultrasonic image signal corresponding to the viewpoint at which the contrast agent is injected into the object.

The controller may be configured to derive the image of the object by accumulating the difference between the ultrasonic image signals.

The controller may be configured to assign an identification element to each of the differences between the ultrasonic image signals.

The controller may be configured to form the image of the object based on an absolute value of the difference between the ultrasonic image signals.

The controller may be configured to form the image of the object by ignoring a signal less than a predetermined magnitude among the differences between the ultrasonic image signals.

The controller may be configured to derive volume information of at least a portion of the object based on the difference between the ultrasonic image signals, and to output the volume information to the display.

The controller may be configured to calculate a volume expansion rate of the object based on the difference between the ultrasonic image signals.

In accordance with another aspect of the disclosure, a method of controlling an ultrasonic imaging apparatus includes deriving, by a controller, a difference between ultrasonic image signals including contrast agent signals obtained in a frame before and after at least one viewpoint; forming, by the controller, an image of an object based on the difference between the ultrasonic image signals over time corresponding to the frame before and after at least one viewpoint; and outputting, by the controller, the image of the object to the display.

The at least one viewpoint may include a viewpoint at which a contrast agent is injected into the object.

The method may further include forming, by the controller, a noise removing filter based on the difference between the ultrasonic image signals corresponding to the viewpoint at which the contrast agent is injected into the object.

The method may further include deriving, by the controller, position information of a difference signal of the ultrasonic image signal corresponding to the viewpoint at which the contrast agent is injected into the object.

The forming of the image of the object may include forming the image of the object by accumulating the difference between the ultrasonic image signals.

The method may further include assigning, by the controller, an identification element to each of the differences between the ultrasonic image signals.

The forming of the image of the object may include forming the image of the object based on an absolute value of the difference between the ultrasonic image signals.

The forming of the image of the object may include forming the image of the object by ignoring a signal less than a predetermined magnitude among the differences between the ultrasonic image signals.

The method may further include deriving, by the controller, volume information of at least a portion of the object based on the difference between the ultrasonic image signals, and outputting the volume information to the display.

The deriving of the volume information of at least a portion of the object may include calculating a volume expansion rate of the object based on the difference between the ultrasonic image signals.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a view illustrating an appearance of an ultrasonic imaging apparatus according to exemplary embodiments of the disclosure;
FIG. 2 is a control block diagram of an ultrasonic imaging apparatus according to exemplary embodiments of the disclosure;
FIG. 3 is a control block diagram specifically illustrating a configuration of a main body of an ultrasonic imaging apparatus according to exemplary embodiments of the disclosure;
FIG. 4 is a control block diagram schematically illustrating a configuration of a main body of an ultrasonic imaging apparatus according to exemplary embodiments of the disclosure;
FIG. 5 is a view for describing contents related to a contrast agent image and a tissue signal component, according to exemplary embodiments of the disclosure;
FIG. 6 is a view for describing an operation of injecting a contrast agent according to exemplary embodiments of the disclosure;
FIG. 7 is a view for describing an operation of deriving a difference between ultrasonic image signals according to exemplary embodiments of the disclosure;
FIG. 8 is a view for describing an operation of outputting an image of an object by accumulating a difference between ultrasonic image signals according to exemplary embodiments of the disclosure;
FIG. 9 is a view for describing a noise removing filter according to exemplary embodiments of the disclosure;
FIGS. 10A and 10B are views for describing an operation of assigning an identification element to a difference between ultrasonic image signals according to exemplary embodiments of the disclosure;
FIG. 11 is a view illustrating an operation of outputting volume information of an object to a display according to exemplary embodiments of the disclosure; and
FIGS. 12 and 13 are flowcharts according to exemplary embodiments of the disclosure.

### DETAILED DESCRIPTION

Like reference numerals refer to like elements throughout the specification. Not all elements of embodiments of the disclosure will be described, and description of what are commonly known in the art or what overlap each other in the embodiments will be omitted. The terms as used throughout the specification, such as "∼ part," "∼ module," "∼ member," "∼ block," etc., may be implemented in software and/or hardware, and a plurality of "∼ parts," "∼ modules," "∼ members," or "∼ blocks" may be implemented in a single element, or a single "∼ part," "∼ module," "∼ member," or "∼ block" may include a plurality of elements.

It will be understood that when an element is referred to as being "connected" to another element, it can be directly or indirectly connected to the other element, wherein the indirect connection includes "connection" via a wireless communication network.

Also, when a part "includes" or "comprises" an element, unless there is a particular description contrary thereto, the part may further include other elements, not excluding the other elements.

Further, when it is stated that a layer is "on" another layer or substrate, the layer may be directly on another layer or substrate or a third layer may be disposed therebetween.

It will be understood that, although the terms first, second, third, etc., may be used herein to describe various elements, it should not be limited by these terms. These terms are only used to distinguish one element from another element.

As used herein, the singular forms "a," "an" and "the" are intended to include the plural forms as well, unless the context clearly indicates otherwise.

An identification code is used for the convenience of the description but is not intended to illustrate the order of each step. Each of the steps may be implemented in an order different from the illustrated order unless the context clearly indicates otherwise.

Hereinafter, the operation principles and embodiments of the disclosure will be described with reference to the accompanying drawings.

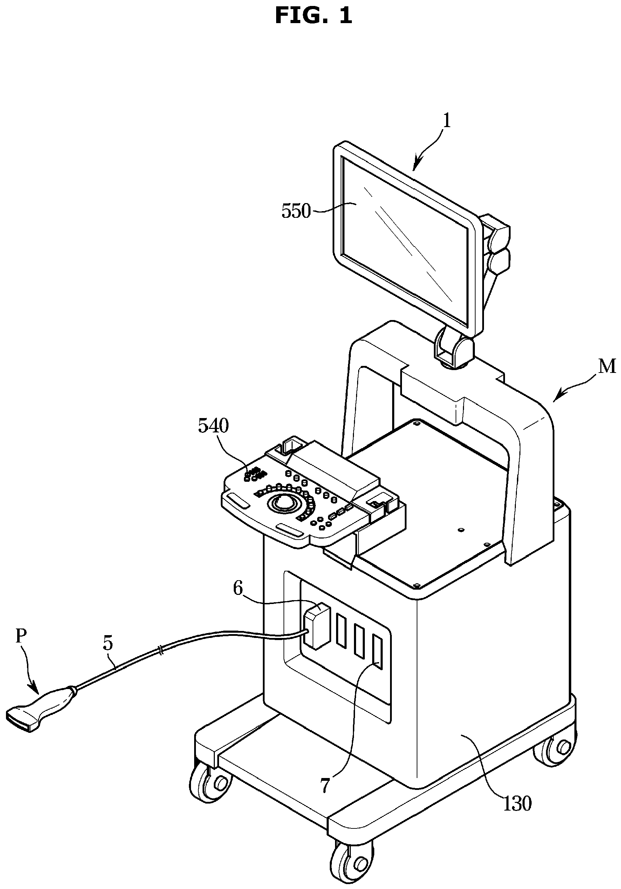

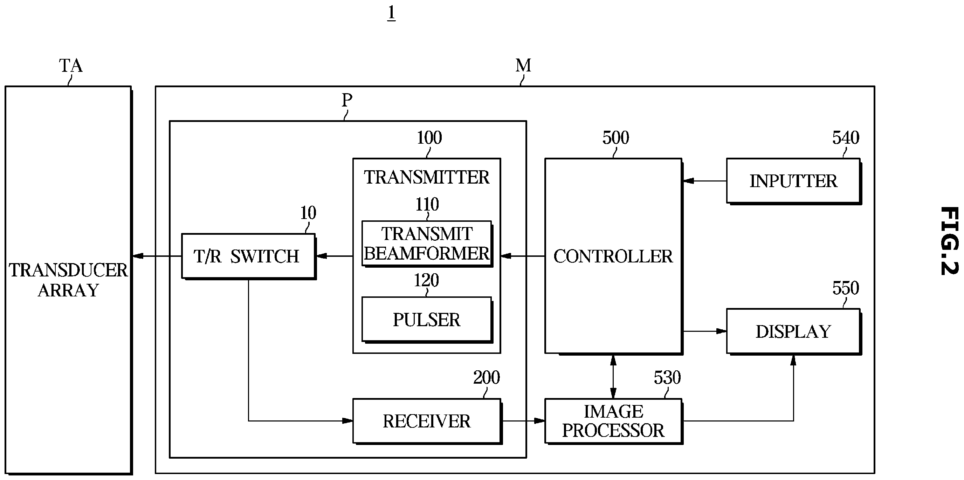

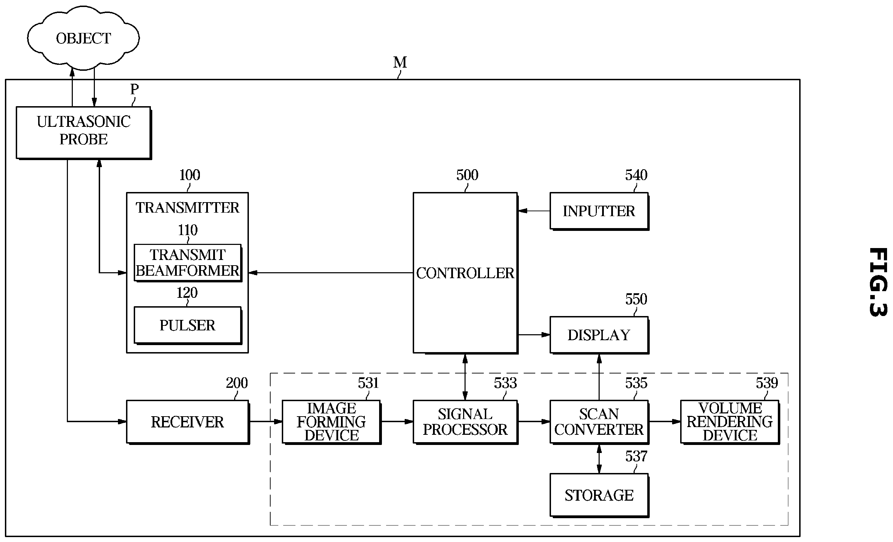

FIG. 1 is a view illustrating an appearance of an ultrasonic imaging apparatus according to exemplary embodiments of the disclosure, FIG. 2 is a control block diagram of an ultrasonic imaging apparatus according to exemplary embodiments of the disclosure, and FIG. 3 is a control block diagram specifically illustrating a configuration of a main body of an ultrasonic imaging apparatus according to exemplary embodiments of the disclosure.

Referring to FIG. 1, an ultrasonic imaging apparatus 1 may include an ultrasonic probe P configured to transmit ultrasonic to an object, receive an ultrasonic echo signal from the object, and convert the received ultrasonic echo signal into an electrical signal; and a main body M connected to the ultrasonic probe P and having an inputter 540 and a display 550 and configured to display an ultrasonic image. The ultrasonic probe P may be connected to the main body M of the ultrasonic imaging apparatus 1 through a cable 5 to receive various signals required for controlling the ultrasonic probe P, or transmit an analog signal or digital signal corresponding to the ultrasonic echo signal received by the ultrasonic probe P to the main body M. However, the embodiment of the ultrasonic probe P is not limited thereto, and the ultrasonic probe P may be implemented as a wireless probe to transmit and receive signals through a network formed between the ultrasonic probe P and the main body M.

The cable 5 may be connected at one end to the ultrasonic probe P and may be provided at the other end with a connector 6 that is coupled to or separated from in a slot 7 of the main body M. The main body M and the ultrasonic probe P may exchange control commands or data using the cable 5. For example, when a user inputs information about a focal depth, a size or shape of an aperture, or a steering angle through the inputter 40, the information is transmitted to the ultrasonic probe P through the cable 5 to thereby be used by a beamforming apparatus (not shown). Alternatively, when the ultrasonic probe P is implemented as a wireless probe as described above, the ultrasonic probe P is connected to the main body M through a wireless network, rather than the cable 5. Even when the main body M is connected to the main body M through a wireless network, the main body M and the ultrasonic probe P may exchange the above-described control commands or data. As illustrated in FIG. 2, the main body M may include a controller 500, an image processor 530, an inputter 540, and a display 550.

The controller 500 may controls overall operations of the ultrasonic imaging apparatus 1. In particular, the controller 500 may generate a control signal for controlling each component of the ultrasonic imaging apparatus 1, for example, a transmitter 100, a T/R switch 10, a receiver 200, an image processor 530, the display 550, and the like illustrated in FIG. 2, and may control the operations of the above-described components. In the ultrasonic imaging apparatus 1 illustrated in FIGS. 2 and 3, a transmission / reception beamformer is included in the ultrasonic probe P rather than the main body M, but the transmission / reception beamformer may be included in the main body M instead of the ultrasonic probe P.

The controller 500 may calculate delay profiles of a plurality of ultrasonic transducer elements 60 constituting an ultrasonic transducer array TA and calculate time delay values in accordance with distance differences between each of the plurality of ultrasonic transducer elements 60 included in the ultrasonic transducer array TA and a focal point of the object based on the calculated delay profiles. In addition, the controller 500 may control the transmission / reception beamformer in accordance therewith to generate transmission / reception signals.

The transducer array TA may be configured to be included in the main body M or the ultrasonic probe P probe P.

Also, the controller 500 may control the ultrasonic imaging apparatus 1 by generating control commands for the respective components of the ultrasonic imaging apparatus 1 according to a user's instruction or command input through the inputter 540.

The image processor 530 may generate an ultrasonic image of a target portion inside the object based on ultrasonicsignals focused by the receiver 200.

Referring to FIG. 3, the image processor 530 may include an image forming device 531, a signal processor 533, a scan converter 535, a storage 537, and a volume rendering device 539.

The image forming device 531 may generate a coherent two-dimensional (2D) image or three-dimensional (3D) image of the target portion inside the object based on the ultrasonic signals focused by the receiver 20.

The signal processor 533 may convert information on the coherent image generated by the image forming device 531 into ultrasonic image information according to a diagnosis mode, such as a brightness mode (B-mode) or a Doppler mode (D-mode). For example, when the diagnosis mode is set to the B-mode, the signal processor 533 may perform and analog/digital (A/D) conversion process, or the like and generate ultrasonic image information for a B-mode image in real time. Alternatively, when the diagnosis mode is set to the D-mode, the signal processor 533 may extract information on phase changes from the ultrasonic signal, calculate information on a blood stream corresponding to each point of cross-sectional image such as speed, power, and distribution, and generates ultrasonic image information for a D-mode image in real time.

The scan converter 535 may convert the converted ultrasonic image information received from the signal processor 533 and the converted ultrasonic image information stored in the storage 537 into general video signals for the display 550 and transmit the converted signals to the volume rendering device 539.

The storage 537 may temporarily or non-temporarily store the ultrasonic image information converted by the signal processor 533.

The volume rendering device 539 may perform volume rendering based on the video signals received from the scan converter 535, correct rendered image information to generate a final resultant image, and transmit the generated resultant image to the display 550.

The inputter 540 allows the user to input a command related to the operation of the ultrasonic imaging apparatus 1. The user may input or set an ultrasonic diagnosis start command, a diagnosis mode select command to select the B-mode, a motion mode (M-mode), the D-mode, an elastography mode (E-mode), or a 3D-mode, region of interest (ROI) setting information including size and position of a ROI, and the like through the inputter 540.

The B-mode image may refer to an image displaying the cross-section of the inside of the object and portions with strong echo signals are distinguished from portions with weak echo signals by modulating brightness. The B-mode image is generated based on information obtained from tens to hundreds of scan lines.

The M-mode may refer to an image representing changes over time in biometric information (e.g., brightness information) on a particular portion (M line) in a cross-sectional image (B-mode image). In general, the B-mode image and a M-mode image are simultaneously displayed on one screen to allow to the user to accurately diagnose by comparing and analyzing the two types of data.

The D-mode image may refer to an image of a moving object obtained by the Doppler effect in which a frequency of sound emitted from a moving object changes. Modes using the Doppler effect may further be classified into a power Doppler imaging (PDI) mode, a color flow (S Flow) mode, and a directional power Doppler imaging (DPDI) mode.

A PDI mode image may refer to an image representing the degree of Doppler signal or the number of structures (number of erythrocytes in blood). In the PDI mode, there is no aliasing signals due to less sensitivity to an angle of incidence and image attenuation caused by noise decreases. Also, since reflected Doppler energy is recorded, the PDI mode is very sensitive enabling detection of small blood vessels and blood streams with low speed.

The S Flow mode may provide a power image (PDI) representing the power of a Doppler signal in 2D distribution and a velocity image representing the velocity of the Doppler signal in 2D distribution. A S flow image may not only visualize blood streams in real time but also represent a wide range of blood stream statuses from a high velocity blood stream in a larger blood vessel to a low velocity blood stream in a smaller blood vessel.

A DPDI mode image may refer to a directional image representing information on a direction of a Doppler signal in 2D distribution in the PDI mode. Thus, the DPDI mode may detect information on blood streams more accurately than the PDI mode. In addition, the M-mode image may be generated in the D-mode.

The E-mode may refer to a method of obtaining an ultrasonic elastography image by using elastography. In this regard, elastography refers to an analysis of a phenomenon in which elasticity of tissues decreases in a hard structure such as malignant mass, and thus the degree of deformation of the tissues by pressure decreases. An ultrasonic elastography image refers to an image quantitatively representing stiffness of tissues. Particularly, the E-mode has been widely used in diagnosis of cervix cancer, breast cancer, or prostate cancer.

A 3D-mode image may refer to an image representing a geometric conformation or a space including X, Y, and Z values respectively representing depth, width, and height or a series of images indicating a stereoscopic feeling as a 3D shape or providing a stereoscopic effect. For example, the user may display a face shape of a fetus by using stereoscopic effects of the 3D-mode and provide parents of the fetus with the face shape.

Meanwhile, the operation of the disclosure may be performed in an ultrasonic contrast agent (UCA) image obtained by entering an ultrasonic contrast agent (UCA) image mode, but the disclosure is not limited to the corresponding mode, and the disclosure is not limited as long as the image of the object is derived based on the difference in the image signal.

The ultrasonic imaging apparatus 1 may operate in the B-mode for obtaining a tissue image, a low voltage B-mode for obtaining a contrast agent image and the tissue image simultaneously, a contrast agent image mode for obtaining the contrast agent image, and the contrast agent image mode prior to administration of a contrast agent.

The contrast agent image described herein may be defined as a technique for imaging by using characteristics that the echo signal reflected from microbubbles constituting the ultrasonic contrast agent (UCA) is displayed as a strong signal compared to a general tissue. A detailed description thereof will be described later.

The inputter 540 may include various devices allowing the user to input data, instructions, and commands, such as a keyboard, a mouse, a trackball, a tablet, or a touch screen module.

The display 550 may display a menu or information required for ultrasonic diagnosis, an ultrasonic image obtained during an ultrasonic diagnosis process, and the like. The display 550 may display an ultrasonic image of a target portion inside the object generated by the image processor 530. The ultrasonic image displayed on the display 550 may be a B-mode ultrasonic image, an E-mode ultrasonic image, or a 3D ultrasonic image. The display 550 may display various ultrasonic images obtained according to the afore-mentioned modes.

The display 550 may be implemented using various known displays such as a cathode ray tube (CRT) and a liquid crystal display (LCD).

The ultrasonic probe P may include the transducer array TA, the T/R switch 10, the transmitter 100, and the receiver 200 as illustrated in FIG. 2. The transducer array TA may be provided at one end of the ultrasonic probe P. The ultrasonic transducer array TA may refer to a one-dimensional (1D) or 2D array of a plurality of ultrasonic transducer elements 60. While the ultrasonic transducer array TA oscillates by pulse signals or alternating currents supplied thereto, ultrasonic is generated. The generated ultrasonic may be transmitted to the target portion inside the object. In this case, the ultrasonic generated by the ultrasonic transducer array TA may also be transmitted to a plurality of target portions inside the object. In other words, the generated ultrasonic may be multi-focused and transmitted to the plurality of target portions.

The ultrasonic generated by the ultrasonic transducer array TA may be reflected by the target portion inside the object and then return to the ultrasonic transducer array TA. The ultrasonic transducer array TA may receive ultrasonic echo signals returning after being reflected by the target portion. When an ultrasonic echo signal arrives at the ultrasonic transducer array TA, the ultrasonic transducer array TA may oscillate at a predetermined frequency corresponding to a frequency of the ultrasonic echo signal and output an alternating current having a frequency corresponding to the oscillation frequency. Thus, the ultrasonic transducer array TA may convert the received ultrasonic echo signal into an electric signal. Since each of the ultrasonic transducer elements 60 may output an electric signal by receiving the ultrasonic echo signal, the ultrasonic transducer array TA may output electric signals of a plurality of channels.

The ultrasonic transducer may be implemented using a magnetostrictive ultrasonic transducer using a magnetostrictive effect of a magnetic material, a piezoelectric ultrasonic transducer using a piezoelectric effect of a piezoelectric material, or a capacitive micromachined ultrasonic transducer (cMUT) that receives ultrasonic using oscillation of hundreds or thousands of micromachined thin films. In addition, any other types of transducers capable of generating ultrasonic in accordance with electric signals or generating electric signals in accordance with ultrasonic may also be used as the ultrasonic transducer.

For example, the transducer elements 60 may include a piezoelectric vibrator or a thin film. When an alternating current is supplied from a power source, the piezoelectric vibrator or the thin film vibrates at a predetermined frequency in accordance with the supplied alternating current and generates ultrasonic having the predetermined frequency in accordance with the vibration frequency. On the contrary, when an ultrasonic echo signal having a predetermined frequency arrives at the piezoelectric vibrator or the thin film, the piezoelectric vibrator or the thin film vibrates in accordance with the ultrasonic echo signal and outputs an alternating current of a frequency corresponding to the vibration frequency.

The transmitter 100 may apply transmit purses to the transducer array TA to control the transducer array TA to transmit ultrasonic signals to the target portion inside the object. The transmitter 100 may include a transmit beamformer and a pulser. A transmit beamformer 110 may generate a transmit signal pattern in accordance with a control signal of the controller 500 of the main body M and outputs the transmit signal pattern to a pulser 120. The transmit beamformer 110 may generate the transmit signal pattern based on a time delay value of each of the ultrasonic transducer elements 60 constituting the transducer array TA calculated by the controller 500 and transmit the generated transmit signal pattern to the pulser 120.

The receiver 200 may perform a predetermined processing on ultrasonic echo signals received by the transducer array TA and performs receive beamforming. The receiver 200 may include a receive signal processor and a receive beamformer.

The receiver 200 may perform image processing and signal processing after receiving a signal from the transducer. The electric signals converted by the transducer array TA are input to the receive signal processor. The receive signal processor may amplify the electric signals converted from the ultrasonic echo signals before processing the electric signals or performing time delay processing on the electric signals and may adjust gains or compensate attenuation according to depth. More particularly, the receive signal processor may include a low noise amplifier (LNA) to reduce noise of the electric signals received from the ultrasonic transducer array TA and a variable gain amplifier (VGA) to control gain values in accordance with the input signals. The VGA may be, but is not limited to, a time gain compensator (TGC) to compensate gains in accordance with distance from the focal point.

The receive beamformer may perform beamforming for the electric signals received from the receive signal processor. The receive beamformer increases intensities of the signals received from the receive signal processor through superposition. The electric signals beamformed by the receive beamformer are converted into digital signals by an A/D converter and transmitted to the image processor 530 of the main body M. When the main body M includes the A/D converter, analog signals beamformed by the receive beamformer may also be transmitted to the main body M and converted into digital signals in the main body M. Alternatively, the receive beamformer may be a digital beamformer. The digital beamformer may include a storage to sample analog signals and store the sampled signals, a sampling period controller to control a sampling period, an amplifier to adjust a sample size, an anti-aliasing low pass filter to prevent aliasing before sampling, a bandpass filter to select a desired frequency band, an interpolation filter to increase a sampling rate while performing beamforming, a high-pass filter to remove a direct current (DC) component or a low frequency band signal, and the like.

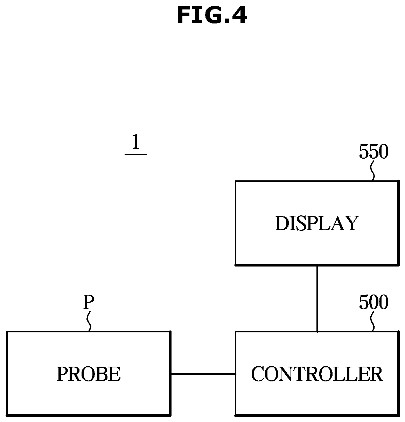

FIG. 4 is a control block diagram schematically illustrating a configuration of a main body of an ultrasonic imaging apparatus according to exemplary embodiments of the disclosure.

Referring to FIG. 4, the ultrasonic imaging apparatus 1 may include, the ultrasonic probe P, the display 550, and the controller 500.

As described above, the probe P may transmit ultrasonic waves to the object, receive the ultrasonic echo signals from the object, and convert the received ultrasonic echo signal into the electrical signal.

As described above, the display 550 may display the menu or information required for ultrasonic diagnosis, the ultrasonic image obtained during the ultrasonic diagnosis process, and the like. The details of the probe P and the display 550 will be omitted.

The controller 500 may derive the difference between ultrasonic image signals obtained in at least one frame before and after at least one viewpoint. In detail, when a plurality of consecutive image frames are obtained, the difference between the ultrasonic image signals may be derived by imaging the difference between the frames.

The controller 500 may control to output the image of the object to the display 550 by forming the image of the object based on the difference between the ultrasonic image signals. A detailed description thereof will be described later.

In addition, in obtaining images of frames before and after the viewpoint, the viewpoint may include a viewpoint of injecting the contrast agent into the object.

The controller 500 may form a noise removing filter based on the difference between the ultrasonic image signals corresponding to the viewpoint at which the contrast agent is injected into the object. The controller 500 may form the noise removing filter based on a difference between the frame obtained before the point in time when the contrast agent is injected into the object and a first frame image obtained after the injection.

The controller 500 may derive position information of a difference signal of the ultrasonic image signal corresponding to the viewpoint at which the contrast agent is injected into the object. That is, in forming the noise removing filter, the position information of the noise may be derived and the noise removing filter may be formed based on the information.

The controller 500 may derive the image of the object by accumulating the difference between the ultrasonic image signals. A detailed operation of the controller 500 accumulating the difference between the ultrasonic image signals will be described later.

The controller 500 may assign an identification element to each of differences between the ultrasonic image signals. The identification element may be given in color, but is not limited thereto and may include an element for identifying a difference from the accumulated image.

The controller 500 may form an image of the object based on an absolute value of the difference between the ultrasonic image signals. In detail, when a negative signal value is derived from a difference image because a signal existing in the frame obtained before the viewpoint does not exist in the frame after the viewpoint, the absolute value of the corresponding signal value may be used or converted a positive signal value by applying an reversion or offset to reflect the difference between the ultrasonic image signals.

The controller 500 may form the image of the object by ignoring a signal less than a predetermined magnitude among the differences between the ultrasonic image signals.

When the difference between the ultrasonic image signals is obtained, the controller 500 may selectively remove or mitigate when some of the signals that are not completely removed by the difference among the other than a contrast agent signal have an intensity less than the predetermined magnitude.

The controller 500 may derive volume information of at least a part of the object based on the difference between the ultrasonic image signals and output the volume information to the display 550.

The volume information may include a volume itself of the object and a volume expansion rate of the object.

In particular, the controller 500 may determine whether the part of the object is blocked using the volume expansion rate.

The controller 500 may apply a motion correction algorithm to compensate for a motion that may occur when obtaining the frame image.

The controller 500 may automatically reproduce and output an image composed of each sum image, or manually move and output a cumulative value of each ultrasonic image difference by a user's manipulation through the inputter.

The controller 500 may divide an outputter into different regions, and may output an image configured as the cumulative value of each ultrasonic image difference in one of the regions. The controller 500 may output the ultrasonic image difference corresponding to the same viewpoint as the image configured as the cumulative value of the ultrasonic image difference shown in the video in other areas.

In the above-described controller 500, the calculation region may be designated by the user as the ROI, or may be automatically designated using an image processing technique such as segmentation.

Meanwhile, the controller 500 may store the difference, the cumulative value, and the like of the ultrasonic image derived in the above-described operation in the storage 537.

The controller 500 may be implemented using a memory (not shown) that stores data on algorithms to control the operation of components of the ultrasonic imaging apparatus 1 or programs to run the algorithms and a processor (not shown) that performs the aforementioned operation by using data stored in the memory. In this case, the memory and the processor may be implemented as separate chips. Alternatively, the memory and the processor may be implemented as a single chip.

At least one component may be added or deleted corresponding to performance of the components of the ultrasound imaging apparatus illustrated in FIG. 4. In addition, it will be readily understood by those skilled in the art that mutual positions of the components may be changed to correspond to performance or structure of a system.

Meanwhile, each of the components illustrated in FIG. 4 may be a software and/or hardware component such as a Field Programmable Gate Array (FPGA) and an Application Specific Integrated Circuit (ASIC).

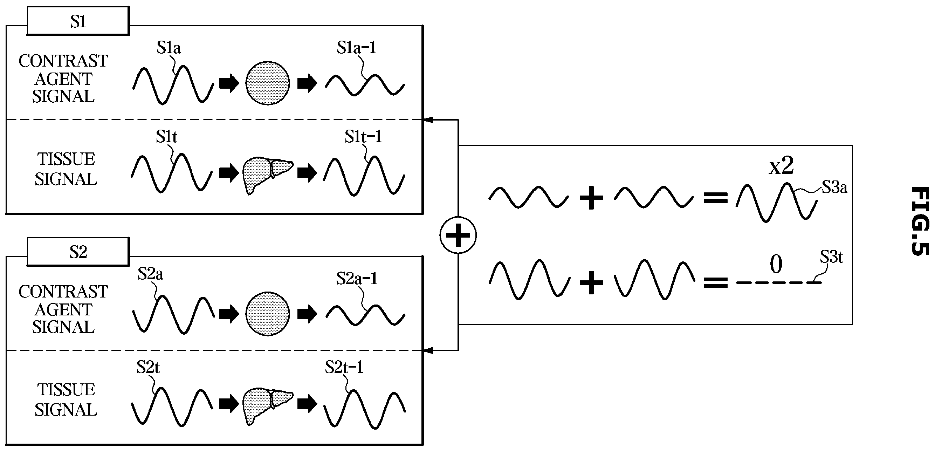

FIG. 5 is a view for describing contents related to a contrast agent image and a tissue signal component, according to exemplary embodiments of the disclosure.

Referring to FIG. 5, the contrast agent image may be defined as a technique of imaging echo signals S1a-1 and S2a-1 reflected from the microbubbles constituting the ultrasonic contrast agent using characteristics that are displayed as the strong signal compared to the general tissue. To this end, general tissue signals S1t-1 and S1t-2 in the image may be minimized so as to be differentiated from the transmission / reception technique of the tissue image, and the transmission / reception technique may be used to highlight the contrast agent signal. Unlike human tissues, the microbubbles exhibit non-linear characteristics that resonate at specific frequencies, and may generate relatively larger echo signals than tissues when ultrasonic signals corresponding to resonance frequencies are applied.

On the other hand, since the microbubbles are destroyed in a high voltage environment of a general tissue image, a signal having a lower voltage than a tissue image transmission signal may be used so that the microbubbles are not destroyed in a human body, and below a specific voltage, while non-linear characteristics in which a harmonic signal is generated in the tissue signals S1t-1 and S2t-1 appear, whereas there may exist a section reacting with linear characteristics in the tissue signals S1t-1 and S2t-1.

In the contrast agent image mode, the tissue signal may be removed using the non-linear characteristics as described above, and only the contrast agent signal S3a may be imaged. Referring to FIG. 5, in a specific voltage condition, when two waveforms S1 and S2 having a phase difference of 180 degrees are transmitted and the receiving component is added, the linear component S3t of the tissue is canceled, and only the non-linear component S3a of the contrast agent remains, so that it can be imaged.

As such, in a specific low voltage condition in which the contrast agent is injected into the object and the contrast agent microbubbles is not destroyed, the transmission and reception mode designed to image the ultrasonic signal by applying the ultrasonic signal to the contrast agent signal S3a and effectively removing the tissue signal S3t may be referred to as the contrast agent image mode. An image obtained in the contrast agent image mode may be defined as the contrast agent image.

In the general tissue image, a cross-sectional image of the inside of the object to be scanned is expressed by the difference between the brightness of the strong and the weak echo, and the transmission / reception technique for this may image a cross section of the tissue using the point that the tissue signal has the linear characteristics at the specific frequency. According to an embodiment, a mode for obtaining the general tissue image may be referred to as the B-mode, and the above image may be defined as the general tissue image.

On the other hand, the tissue image may be separately provided to refer to a tissue signal classification of a current scan position where the user obtains the contrast agent image. To this end, the images may be transmitted and received by alternating frequencies for using the non-linear characteristics of the contrast agent image and frequencies for using the linear characteristics of the tissue signal. This may be divided into one screen and displayed on the display 550, and two screens may be displayed on the display 550 according to the user's selection. In the high voltage environment of B-mode for obtaining the general tissue image, since the microbubbles are destroyed, the linear component generated from the tissues are imaged in a lower voltage environment than the general tissue image. In this way, the transmission / reception mode of a general mode image in the specific voltage environment in which the microbubbles are not destroyed may be defined as a low voltage tissue image, and may be distinguished from the general tissue image. The low voltage tissue image described above may be obtained in an ultrasonic imaging apparatus operating mode defined as the low voltage B-mode which is less than the voltage in the B-mode.

Tissue signal components in the contrast agent image may not be distinguished by themselves, and may be recognized with reference to the low voltage tissue image. After the contrast agent is injected into the object, the low voltage tissue image may include the contrast agent signal, but since the contrast agent is distributed in microvessels in the tissue and overlaps with the tissue, the contrast agent is canceled from the tissue signal so as not to be influenced or emphasized, so that the contrast agent may be referred to the tissue signal classification of the contrast agent image.

Meanwhile, some tissue signal components may be included in the contrast agent image. Due to various traits of the human body, some of human tissue signals may react non-linearly like the contrast agent signal. In this case, the contrast agent signal and the tissue signal cannot be distinguished from the signal point of view in the contrast agent image mode. As a part of the contrast agent signal may be displayed in the contrast agent image.

This is because a part of the tissue signal that has a non-linear response to the object may be seen as the contrast agent signal in the contrast agent image, and may affect diagnostic performance. The image may be referred to as a contrast agent noise image, and may be distinguished from the general tissue image.

On the other hand, the part of the tissue signal that performs a non-linear response to the image obtained in the contrast agent image mode before administration of the contrast agent may be included. That is, because the contrast agent is not included and the non-linear signal is not received, the image obtained in the contrast agent image mode before contrast agent administration may not be obtained.

However, in contrast agent image mode before the contrast agent is administered, the part of the human tissue signals may obtain a non-linear reaction such as the contrast agent signal, and the ultrasonic imaging apparatus 1 may obtain a noise image based on the signal. Meanwhile, in order to distinguish an operation mode in which the above-described operation may be performed from the contrast agent image mode, it may be referred to as the contrast agent image mode before the contrast agent is administered.

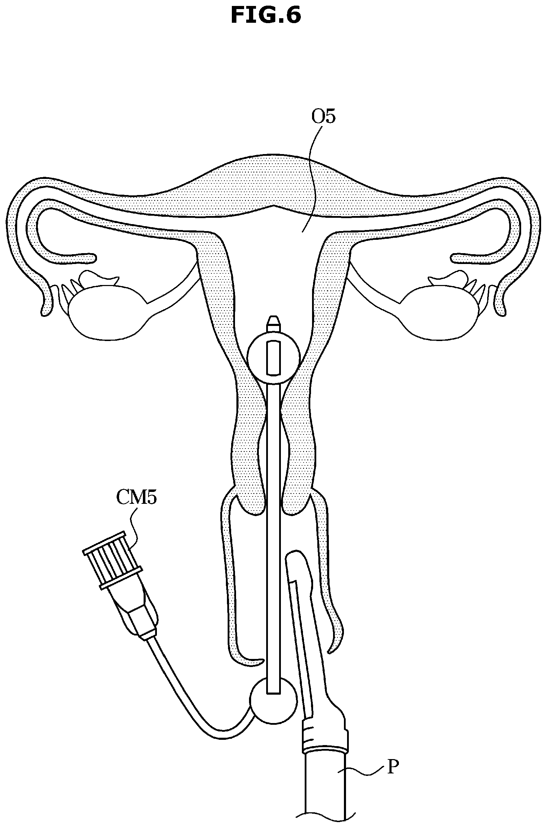

FIG. 6 is a view for describing an operation of injecting a contrast agent according to exemplary embodiments of the disclosure.

Referring to FIG. 6, in an embodiment of the disclosure, an ultrasonic contrast agent image diagnosis method, a test called Hystero-Salpingo Contrast Sonography (HyCoSy) which is performed as an initial test for infertility diagnosis by observing the flow of the contrast agent and diagnosing the opening of fallopian tubes, are proposed. The HyCoSy may be performed in 2D, 3D, and 4D modes, and recently, it is possible to observe the flow of contrast agent CM5 in real time to diagnose tubular opening.

The user controls the ultrasonic imaging apparatus 1, enters the contrast agent image mode, and injects the contrast agent CM5. The flow of the contrast agent image in the image observed by the ultrasonic imaging apparatus 1 may be stored in a frame unit of a specific value per second.

The ultrasonic imaging apparatus 1 may continuously play the stored image frame as if watching a video. The user may observe the contrast agent flowing in the direction of the vagina, uterine lumen and fallopian tubes over time through the ultrasonic imaging apparatus 1.

Meanwhile, in FIG. 6, the method of injecting the contrast agent into the uterine lumen O5 has been described. However, embodiments of the disclosure are not limited thereto, and the method may be applied as long as it can be diagnosed by injecting the contrast agent.

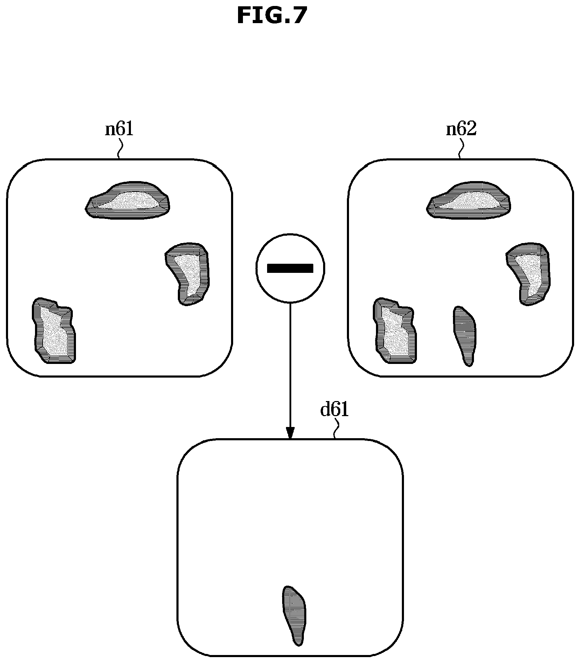

FIG. 7 is a view for describing an operation of deriving a difference between ultrasonic image signals according to exemplary embodiments of the disclosure.

Referring to FIG. 7, FIG. 7 illustrates an image n61 obtained in a frame before injecting the contrast agent and an image n62 obtained in a frame after injecting the contrast agent, and illustrates an image signal difference d61 of two ultrasonic images.

According to an embodiment, the user may scan the object without injecting the contrast agent. As a result of the scan, the controller 500 may obtain the image n61 obtained in the frame before injecting the contrast agent. The controller 500 may derive an image including noises in the shape and position of n61 even though the contrast agent is not injected into the object. Meanwhile, the controller 500 may form the noise removing filter by deriving the position information of the noise of the image n61 obtained in the frame before injecting the contrast agent.

The user injects the contrast agent and then scans the contrast agent signal. At this time, the controller 500 may perform motion correction for the purpose of correcting fine movements of the probe P.

On the other hand, the controller 500 may derive an ultrasonic image signal difference d61 between the two images based on the image n61 obtained in the frame before injecting the contrast agent and the image n62 obtained in the frame after injecting the contrast agent.

The image n61 obtained in the frame before injecting the contrast agent and the image n62 obtained in the frame after injecting the contrast agent may include signals related to noise in common and may differently include the signal according to the presence or absence of the contrast agent over time. Accordingly, the difference d61 between the two image signals may include only the image signal obtained by removing an existing noise signal and being differently obtained according to the flow of the contrast agent. Through this operation, the controller 500 may obtain the ultrasonic image signal from which the noise is removed before injecting the contrast agent.

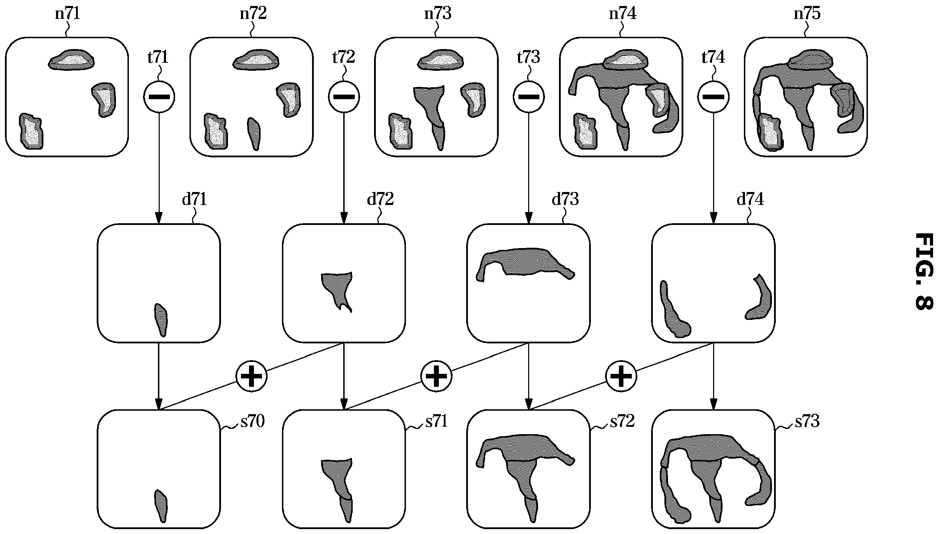

FIG. 8 is a view for describing an operation of outputting an image of an object by accumulating a difference between ultrasonic image signals according to exemplary embodiments of the disclosure.

FIG. 8 illustrates the operation of FIG. 6 and subsequent operation of the controller 500.

Referring to FIG. 8, the controller 500 may obtain ultrasonic image signals n71 to n75 obtained by the probe P. Each ultrasonic image signal may be provided as the ultrasonic image signal obtained in the frame before and after each of the points of time t71 to t74 in chronological order.

The controller 500 may derive differences d71 to d74 of the ultrasonic image signal corresponding to each of the points of time t71 to t74. Since the signal related to noise in the ultrasonic image signal is fixed or there is a high probability that the flow is low, the differences d71 to d74 of each ultrasonic image signal may include the ultrasonic image signal based on the flow of the contrast agent from which the noise signal is removed.

On the other hand, in order to derive the difference between the ultrasonic image signals, since there is no the contrast agent signal at the corresponding position, when the negative signal value is derived from the difference between the ultrasonic image signals, it may be applied to the difference between the ultrasonic image signals by converted to the positive signal value by performing a method such as inversion or offset application.

In addition, when deriving the difference between the ultrasonic image signals, the controller 500 may selectively remove or mitigate when some of the signals that are not completely removed by the difference among the other than a contrast agent signal have the intensity less than the predetermined magnitude.

The controller 500 may accumulate ultrasonic image signal differences d71 to d74 according to the injection flow of the contrast agent, and may sequentially obtain images s70 to s73 of the objects. The controller 500 may sequentially configure and store the images s70 to s73 of each object as one video data.

According to another embodiment, when obtaining the difference between the volume images in the contrast agent image, the controller 500 may generate a single volume by calculating a difference between 2D images having the same position among the frames constituting each volume for the two volume images generated in a certain time zone.

The controller 500 may also calculate the difference between two volume images generated at any time.

The controller 100 may output the video through the display 550 of the ultrasonic imaging apparatus 1.

On the other hand, the above operation is only an embodiment for describing embodiments of the disclosure, there is no limitation in the operation of deriving the image of the object by accumulating the difference between the ultrasonic image signals.

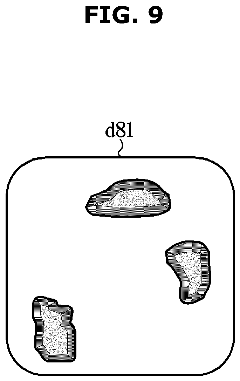

FIG. 9 is a view for describing a noise removing filter according to exemplary embodiments of the disclosure.

Referring to FIG. 9, the controller 500 may derive the difference between ultrasonic image signals obtained in at least one frame before and after at least one viewpoint. The at least one viewpoint may include a viewpoint at which the contrast agent is injected into the object.

FIG. 9 illustrates a difference d81 of the ultrasonic image signal obtained in the frame before and after the viewpoint at which the contrast agent is injected into the object. The controller 500 may derive the difference between the ultrasonic image signals obtained in the frames before and after the various points of time, but in particular, the difference d81 of the ultrasonic image signals obtained in the frames before and after injecting the contrast agent may include the noise before injecting the contrast agent.

The noise before injecting the contrast agent may result from gas in the human body, strong reflection signals from the tissue, residual contrast agent injected from a previous scan, and the like.

Meanwhile, the difference d81 of the ultrasonic image signal illustrated in FIG. 9 may be derived based on the difference between the ultrasonic image signals of the frame before and after injecting the contrast agent time and may include the noise signal described above.

The controller 500 may store the difference between the ultrasonic image signals of the frame before and after injecting the contrast agent in the storage 537 and use it as the noise removing filter.

The Noise removing filter may use identification elements to differentiate them from ultrasonic image signals.

The noise removing filter may be configured with a color that is distinguished from the ultrasonic image of the existing object, but is not limited to any color as long as it can be distinguished from the ultrasonic image of the existing object. For example, the identification element may include color, shade, and pattern.

The controller 500 may obtain the image of the object by accumulating the difference between the ultrasonic image signals based on the above-described method, and may remove the noise using the noise removing filter.

In other words, the controller 500 may obtain the ultrasonic image according to the injection of the contrast agent and obtain the image of the object from which the noise is removed by applying the noise removing filter to the obtained image.

Meanwhile, FIG. 9 is an example of the noise removing filter. When the noise removing filter is based on the difference between the ultrasonic image signals obtained in the frame before and after injecting the contrast agent, the implementation is not limited.

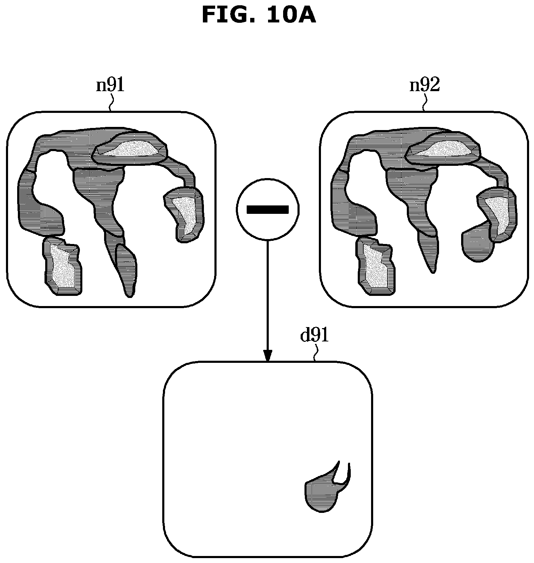

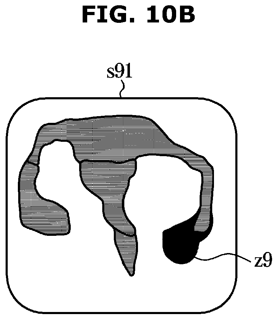

FIGS. 10A and 10B are views for describing an operation of assigning an identification element to a difference between ultrasonic image signals according to exemplary embodiments of the disclosure.

FIG. 10A, the controller 500 may assign the identification element to a difference d91 between the ultrasonic image signals. The identification element may be configured with the color that is distinguished from the ultrasonic image of the existing object, but is not limited to any color as long as it can be distinguished from the ultrasonic image of the existing object. For example, the identification element may include color, shade, and pattern.

Referring to FIG. 10B, the difference between the ultrasonic image signals including the identification element in the image of the object is distinguished from the image of the object and is displayed on the display 550. In outputting the image of the object, the controller 500 may assign the identification element to an additional portion, that is, the ultrasonic image difference, over time, and may output the image on the display 550 so that it can be distinguished from the image of the object derived up to now.

In detail, the controller 500 may obtain the ultrasonic image in real time and display the result of accumulating the difference between the ultrasonic contrast agent images on the display 550, and may output the image accumulating the image signal difference separately on the display 550 after all the scanning processes are completed.

For example, in a patient with a blocked end of one fallopian tube, when the injected contrast agent flows to the blocked end of the fallopian tube and there is no room for further flow, the injected contrast agent concentrates at the end of the fallopian tube over time, causing fallopian tube to expand.

The controller 500 may obtain the ultrasonic image obtained at the viewpoint before the expansion of the fallopian tube, the ultrasonic image obtained at the viewpoint after the expansion of the fallopian tube, and the ultrasonic image with a flow of the contrast agent of the opposite fallopian tube.

The controller 500 may compare each ultrasonic image to expand the fallopian tube and there is a change in the signal of the fallopian tube region. Thus, the controller 500 may generate the changed image by displaying the expanded region in the color and pattern different from the existing contrast agent image.

On the other hand, the operation described in FIG. 10 is only an example for describing the operation of the disclosure. When the identification element is assigned to the difference between the ultrasonic images obtained over time as long as it can be distinguished from the existing object image, and there is no limitation of the element.

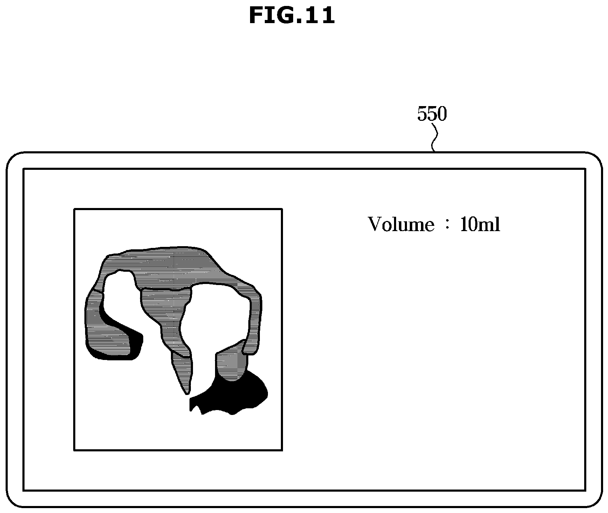

FIG. 11 is a view illustrating an operation of outputting volume information of an object to a display according to exemplary embodiments of the disclosure.

The controller 500 may derive the image of the object in the above-described methods, and may derive volume information of at least a part of the object from the derived image of the object. The following method may be used by the controller 500 to derive the volume information of the object.

To derive the volume information of the object, a region for deriving the volume information may be designated.

The region for deriving the volume information may be designated by the user, or may be automatically designated using the image processing technique such as segmentation. In addition, when the user designates, the designated region may be selected by figure or free form. Meanwhile, the figure may include a polygon, a circle, an ellipse, a rectangle, and the like. The controller 500 may trace the object's outline on all cross sections, and then calculate the volume of the object by calculating and considering the region of each cross section.

In another embodiment, the controller 500 may trace the outline only for the maximum cross section, calculate its region, assume the shape of the organ as the ellipse, and calculate the volume when the maximum cross section is rotated about a long axis with a predetermined formula.

The controller 500 may calculate the volume of the object by the above-described method, and may also calculate a volume expansion rate of the object using the same to determine whether the part of the object is blocked. Particularly, when the volume expansion ratio of the object exceeds a predetermined value, it may be determined that a part of the object is blocked.

On the other hand, the predetermined value is a value that can be arbitrarily set by the user, and there is no limitation on how to set the predetermined value.

In FIG. 11, the controller 500 outputs the volume of the uterus measured by the above-described method on the display 550. However, the volume information of the object that the controller 500 can derive is not limited.

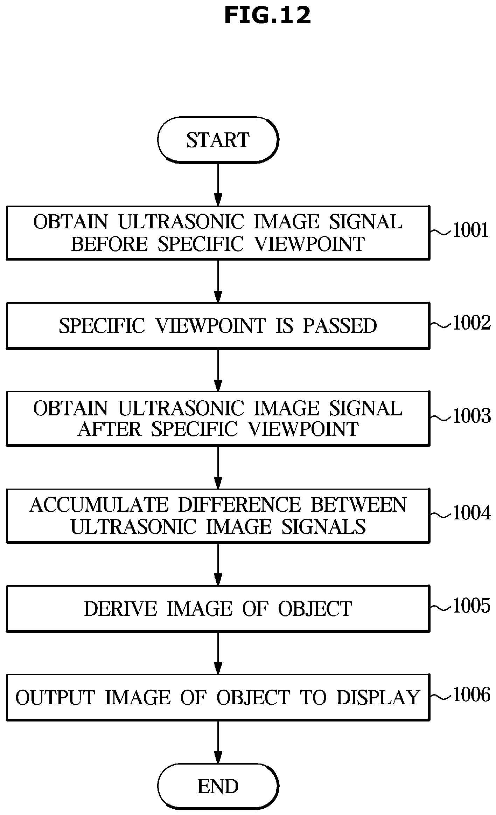

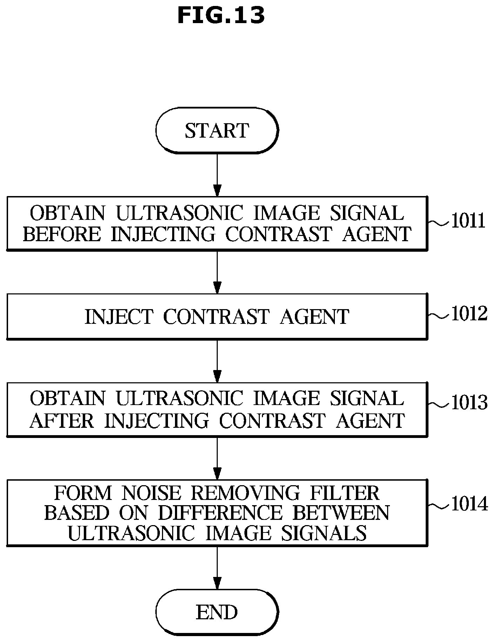

FIGS. 12 and 13 are flowcharts according to exemplary embodiments of the disclosure.

Referring to FIG. 12, the controller 500 may obtain the ultrasonic image signal of the frame before the specific viewpoint (1001), and when the specific viewpoint is passed (1002), the controller 500 may obtain the ultrasonic image signal of the frame after the specific viewpoint (1003). The controller 500 may derive the difference between the ultrasonic image signals before and after the specific viewpoint and accumulate the difference between the ultrasonic image signals (1004), and may derive the image of the object (1005). On the other hand, the controller 500 may output the image of the derived object to the display 550 (1006).

Referring to FIG. 13, the controller 500 may obtain the ultrasonic image signal of the frame before injecting the contrast agent (1011). When the user injects the contrast agent (1012), the controller 500 may obtain the ultrasonic image signal after injecting the contrast agent (1013).

Since the difference between the ultrasonic image signals is the difference between the ultrasonic image signals at the viewpoint of injection of the contrast agent, the controller 500 may form the noise removing filter based on this (1014).

According to the ultrasonic imaging apparatus and the method of controlling the ultrasonic imaging apparatus according to the exemplary embodiments, the image of the object may be obtained by efficiently removing noise from the ultrasonic image obtained using the contrast agent.

Meanwhile, the disclosed exemplary embodiments may be implemented in the form of a recording medium storing instructions that are executable by a computer. The instructions may be stored in the form of a program code, and when executed by a processor, the instructions may generate a program module to perform operations of the disclosed exemplary embodiments. The recording medium may be implemented non-transitory as a computer-readable recording medium.

The non-transitory computer-readable recording medium may include all kinds of recording media storing commands that can be interpreted by a computer. For example, the non-transitory computer-readable recording medium may be, for example, ROM, RAM, a magnetic tape, a magnetic disc, flash memory, an optical data storage device, etc.

Embodiments of the disclosure have thus far been described with reference to the accompanying drawings. It will be obvious to those of ordinary skill in the art that the disclosure may be practiced in other forms than the embodiments as described above without changing the technical idea or essential features of the disclosure. The above embodiments are only by way of example, and should not be interpreted in a limited sense.

## Claims

1. An ultrasonic imaging apparatus comprising:
a display; and
a controller configured to:
derive a difference between ultrasonic image signals including contrast agent signals obtained in a frame before and after at least one viewpoint; and
form an image of an object based on the difference between the ultrasonic image signals over time corresponding to the frame before and after at least one viewpoint and output the image of the object to the display.

2. The ultrasonic imaging apparatus according to claim 1, wherein the at least one viewpoint comprises a viewpoint at which a contrast agent is injected into the object.

3. The ultrasonic imaging apparatus according to claim 2, wherein the controller is configured to form a noise removing filter based on the difference between the ultrasonic image signals corresponding to the viewpoint at which the contrast agent is injected into the object.

4. The ultrasonic imaging apparatus according to claim 2, wherein the controller is configured to derive position information of a difference signal of the ultrasonic image signal corresponding to the viewpoint at which the contrast agent is injected into the object.

5. The ultrasonic imaging apparatus according to claim 1, wherein the controller is configured to derive the image of the object by accumulating the difference between the ultrasonic image signals.

6. The ultrasonic imaging apparatus according to claim 5, wherein the controller is configured to output the difference between the accumulated ultrasonic image signals to the display.

7. The ultrasonic imaging apparatus according to claim 1, wherein the controller is configured to assign an identification element to each of the differences between the ultrasonic image signals.

8. The ultrasonic imaging apparatus according to claim 1, wherein the controller is configured to form the image of the object based on an absolute value of the difference between the ultrasonic image signals.

9. The ultrasonic imaging apparatus according to claim 1, wherein the controller is configured to form the image of the object by ignoring a signal less than a predetermined magnitude among the differences between the ultrasonic image signals.

10. The ultrasonic imaging apparatus according to claim 1, wherein the controller is configured to derive volume information of at least a portion of the object based on the difference between the ultrasonic image signals, and to output the volume information to the display.

11. The ultrasonic imaging apparatus according to claim 10, wherein the controller is configured to calculate a volume expansion rate of the object based on the difference between the ultrasonic image signals.

12. A method of controlling an ultrasonic imaging apparatus comprising:
deriving, by a controller, a difference between ultrasonic image signals including contrast agent signals obtained in a frame before and after at least one viewpoint;
forming, by the controller, an image of an object based on the difference between the ultrasonic image signals over time corresponding to the frame before and after at least one viewpoint; and
outputting, by the controller, the image of the object to the display.

13. The method according to claim 12, wherein the at least one viewpoint comprises a viewpoint at which a contrast agent is injected into the object.

14. The method according to claim 13, further comprising:
forming, by the controller, a noise removing filter based on the difference between the ultrasonic image signals corresponding to the viewpoint at which the contrast agent is injected into the object.

15. The method according to claim 13, further comprising:
deriving, by the controller, position information of a difference signal of the ultrasonic image signal corresponding to the viewpoint at which the contrast agent is injected into the object.
